(19) Europäisches Patentamt
European Patent Office
Office européen des brevets

(11) Publication number: 0 147 476 B1

# (12) EUROPEAN PATENT SPECIFICATION

(45) Date of publication of patent specification: 11.07.90

(51) Int. Cl.$^5$: A 61 K 31/195, A 61 K 47/00

(21) Application number: 83112373.2

(22) Date of filing: 08.12.83

## (54) Gel preparations for external application.

(43) Date of publication of application:
10.07.85 Bulletin 85/28

(45) Publication of the grant of the patent:
11.07.90 Bulletin 90/28

(84) Designated Contracting States:
BE CH DE FR GB IT LI NL SE

(56) References cited:
FR-A-2 500 751
GB-A-2 023 000
US-A-3 524 916

CHEMICAL ABSTRACTS, vol. 98, no. 16, 16th April 1983, page 367, no. 132249w, Columbus, Ohio, USA; K. KYUKI et al.: "Antiinflammatory effect of diclofenac sodium ointment (cream) in topical application" & JPN. J. PHARMCOL. 1983, 33(1), 121-132

The file contains technical information submitted after the application was filed and not included in this specification

(73) Proprietor: Toko Yakuhin Industry Co., Ltd.
12-23, 2-chome, Honjyo-Nishi
Oyodo-ku Osaka (JP)

(72) Inventor: Kamishita, Takuzo
12-28, 6-chome, Tonda-cho
Takatsuki-shi Osaka (JP)

(74) Representative: Kraus, Walter, Dr. et al
Patentanwälte Kraus, Weisert & Partner
Thomas-Wimmer-Ring 15
D-8000 München 22 (DE)

(56) References cited:
H.Janistyn, Handbuch der Kosmetika und Riechstoffe, 1 Band, Heidelberg 1978, pages 154 to 156

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 147 476 B1

Courier Press, Leamington Spa, England.

## Description

The present invention relates to gel preparations for external application containing diclofenac sodium as active ingredient and having good stability and nice feeling on use.

Diclofenac sodium is a derivative of phenylacetic acid represented by the formula:

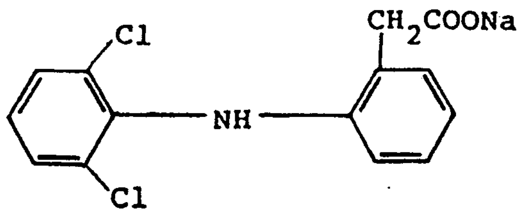

which is a non-steroid drug soluble in water and alcohols having excellent antiinflammatory and analgetic effects.

For the present, it is used only in the form of oral preparations or suppositories and shows excellent antiinflammatory and analgetic effects. However, side effects such as stomach and intestines trouble, liver trouble and kidney trouble, are at issue, especially in the case of the oral administration. Therefore, antiinflammatory and analgetic preparations which are absorbed cutaneously without showing such side effects are desired.

In this regard, a gel preparation for external application containing indomethacin, a non-steroid drug, is known (Japanese Patent Laid-open No. Sho 53(1978)-81616). In this preparation, however, there is a question of stability and there is a defect that its yellow color due to the color of indomethacin itself soils clothes as it is applied on the skin. Thus, the inventor of the present invention has found preparations for external application which consist of a solution comprising another non-steroid compound having antiinflammatory and analgetic effects, at least one of peppermint oil and salicylic acid ester in an amount sufficient to dissolve the non-steroid compound and a base for external application (Japanese Patent Application No. Sho 56(1981)-128032). However, any and every compound having antiinflammatory and analgetic effects, which may be used in such preparations, is limited to water-insoluble one.

In FR-A-2 500 751 it is proposed to use quaternary salts of diclofenac (2-(2,6-dichloroanilino)-phenylacetic acid) other than the sodium salt which is used according to this invention.

CHEMICAL ABSTRACTS, vol. 98, no. 16, 16th April 1983, page 367, no. 132249w, Columbus, Ohio, USA; K. KYUKI et al.: "Antiinflammatory effect of diclofenac sodium ointment (cream) in topical application" & JPN. J. PHARMACOL. 1983, 33(1), 121-132 discloses ointments of diclofenac-Na prepared with three kinds of bases: lipophilic, emulsion (cream) and gel bases. But it specifically discloses only the cream among the above three kinds of ointments and does not disclose the gel.

GB-A-2 023 000 discloses a gel preparation of indomethacin.

US-A-3 524 916 discloses only a preparation for the external treatment of athlete's foot comprising griseofulvin and ethylene glycol monosalicylate.

This invention relates to a gel preparation for external application which is characterized by comprising diclofenac sodium as the active ingredient and present in an amount of 0.3 to 3.0% by weight; a medium comprising water, ethanol, and a glycol selected from the group consisting of ethylene glycol, propylene glycol and 1,3-butylene glycol, the medium being present in an amount at least sufficient to dissolve the active ingredient, the ratio of water to the combination of the ethanol and glycol being from about 8:2 to 4:6; a carboxy-vinyl polymer selected from the group consisting of the hydrophillic polymers obtained by polymerisation of acrylic acid, and present in an amount sufficient to gel the preparation; and an aliphatic amine, present in an amount sufficient to adjust the pH to about 6 to 8.

The gel preparations for external application of this invention have good stability and nice feeling on use and show excellent antiinflammatory and analgetic effects by cutaneous absorption.

Fig. 1 is a graph showing changes with the lapse of time in the amount of diclofenac sodium contained in the preparation of Example 1 of the present invention, and Figs. 2 to 4 are graphs showing changes with the lapse of time in the amount of diclofenac sodium contained in the preparations of Referential Examples 1 to 3, respectively.

The content of diclofenac sodium, the active ingredient, in the gel preparation of the present invention is usually 0.3 to 3.0% by weight, preferably, 0.5 to 2.0% by weight of the preparation.

As the medium for the active ingredient, water, ethanol and glycols are used.

It is possible to prepare a stable gel preparation of diclofenac sodium using as medium a combination of water and ethanol or a combination of water and glycols. However, when water and ethanol are used, absorption of the active ingredient diclofenac sodium is not good, because the preparation gets dry so easily that diclofenac sodium crystallizes out on the surface of the skin. On the other hand, when water and a glycol are used as medium, it is necessary to use the glycol in an amount of at least 30% or more, in consideration of irritation which may be caused by pH on the skin. Nevertheless, if a glycol is used for the gel preparation in an amount of 30% or more, the preparation does not get dry well as applied on the skin. Moreover, the irritation on the skin by the glycol remains still as a problem.

Therefore, it is required to use a combination of water, ethanol and glycols.

The medium in the preparation of the present invention is used in such amount as is sufficient at least to dissolve the active ingredient and as constitutes the whole preparation together with the gelating agent, the neutralizing agent, the active ingredient, etc.

The ratio by weight of water to organic solvents in the medium is from 8:2 to 4:6. It is preferred that water is 80% by weight or less, ethanol is 60% by weight or less and glycols are 40% by weight or less of the medium. As a preferable mixing ratio by weight for the medium use in the present invention, there can be mentioned a ratio of water: ethanol: glycols of approximately 60:30:10.

As the glycols, ethylene glycol, propylene glycol and 1,3-butylene glycol are mentioned. The preferred among these are propylene glycol and 1,3-butylene glycol.

The most preferable combination for the medium of the above three solvents is water: ethanol: propylene glycol or water: ethanol: 1,3-butylene glycol.

Carboxyvinyl polymers used as the gelating agent are hydrophillic polymers obtained by polymerization of acrylic acid as the main component. It is desirable to use a commercial product. As commercially available products, there can be mentioned Hiviswako 103®, Hiviswako 104®, and Hiviswako 105® from Wakojunyakukogyo K.K. in Japan, Carbopol 934®, Carbopol 940® and Carbopol 941® from Goodrich Chemical Co. in U.S.A., and the like.

In the preparations of the present invention, weak basic substances are used as the neutralizing agent in such amount as enables to adjust the preparations almost neutral, that is, at a pH of 6 - 8, preferably, a pH of 6.5 - 7.5. An amount of, for example, 0.1 - 5% by weight of the preparation would suffice to attain the purpose.

As the weak basic substances, aliphatic amines are preferred. The aliphatic amines include primary, secondary and tertiary alkanolamine and primary, secondary and tertiary alkylamines. As concrete examples of the alkanolamines, there can be mentioned monoethanolamine, diethanolamine, diisopropanolamine, triethanolamine, triisopropanolamine, etc. As the alkylamines are mentioned dimethylamine, diethylamine, trimethylamine, triethylamine and the like. Especially preferred among these are triethanolamine and diisopropanolamine. To use such weak basic substance as the neutralizing agent is one of the characteristic features of the present invention. It is proved that to use a strong base such as sodium hydroxide as the neutralizing agent is improper. Comparing carboxyvinyl polymers with diclofenac in the form of free acid, the acidity of the former is stronger than that of the latter. However, carboxyvinyl polymers are by themselves weak acids. Accordingly, when a strong base such as sodium hydroxide is used as the neutralizing agent, the pH value increases to about 9. Although it is possible to prepare gel preparations of diclofenac sodium using a gelating agent under such condition, the strong basicity increased to a pH value of about 9 is undesirable for the preparations for external application because it causes irritation on the skin. On the other hand, when gel preparations of diclofenac sodium are prepared without neutralizing the carboxyvinyl polymer with sodium hydroxide, the free acid of diclofenac is formed in the resulting gel preparations since the sodium ion of diclofenac sodium combines with said carboxyvinyl polymer having an acidity stronger than diclofenac even if the polymer were under the pH condition of about 7. Although diclofenac in the form of sodium salt is stable, diclofenac itself is hardly soluble and loses stability in the preparations with the lapse of time.

Further, to the gel preparation of the present invention, at least one of peppermint oil, l-menthol, methyl salicylate, ethyl salicylate and glycol monosalicylate may be added, if desired. Peppermint oil and l-menthol give cool feeling to the skin, and salicylic acid derivatives accelerate cutaneous absorption of the active ingredient. They are applied as an inductive stimulant in the case of pain complaints and increase the analgetic effects. These auxiliary agents are added to the gel preparations usually in an amount of 0.5 to 5% by weight.

Although there is no limitation on the method of preparing the gel preparation of the present invention, they can be prepared, for example, according to the following methods: Diclofenac sodium is dissolved in ethanol. To the solution obtained are added an aqueous solution of a carboxyvinyl polymer and glycol, while stirring. Then, a neutralizing agent is added further to the solution, while stirring, in such amount as adjusts the pH of the resulting gel preparation at about 6 - 8. Thus, the gel preparation of the present invention is obtained. The gel preparation of the present invention can be prepared also by adding an aqueous solution of a carboxyvinyl polymer to a solution obtained by dissolving diclofenac sodium in a mixture of ethanol and a glycol, while stirring, and then adding a neutralizing agent to the resulting solution while stirring.

To the gel preparation of the present invention, those applicable in the art, such as aromatic agent, antiseptic agent, coloring agent, etc., may be added in a small amount, besides the salicylic acid esters as mentioned above, if so desired. However, good preparations are obtained usually without necessity for adding such additives.

The gel preparation of the present invention thus obtained have good stability. They do not show any change in the viscosity even at high temperatures and any crystallization at low temperatures. Moreover, they adhere well to the skin and spread very well. Further, they do not give sticky feeling and they get dry easily.

In the following, the present invention is illustrated by giving Examples of the preparations of the present invention and Referential Examples of preparations having a similar composition and a low pH value. However, the invention shall not be limited to these Examples.

Example 1

Diclofenac sodium (1g) was dissolved in 95% ethanol (30g) while stirring. On the other hand, propylene glycol (10g), 4% aqueous solution (25g) of a carboxyvinyl polymer Carbopol 940® and purified water (20g) were mixed uniformly by stirring, and triethanolamine (1.5g) was added to the mixture while continuing the stirring. To the gel base thus prepared, the alcoholic solution of diclofenac sodium previously prepared was added and the whole was adjusted to 100g by further adding purified water. After stirring well, a gel preparation having a viscosity of 20,000 centipoise and a pH of 7.15 was obtained.

Example 2

Diclofenac sodium (0.5g) was dissolved in 95% ethanol (25g) while stirring. On the other hand, 1,3-butylene glycol (20g), 4% aqueous solution (25g) of Carbopol 940® and purified water (20g) were mixed uniformly by stirring, and triethanolamine (1.5g) was added to the mixture while continuing the stirring. To the gel base thus prepared, the alcoholic solution of diclofenac sodium previously prepared was added and the whole was adjusted to 100g by further adding purified water. After stirring well, a gel preparation having a viscosity of 22,000 centipoise and a pH of 7.15 was obtained.

Example 3

Diclofenac sodium (3.0g) and l-menthol (0.5g) were dissolved in 95% ethanol (40g) by stirring. On the other hand, propylene glycol (10g), 4% aqueous solution (25g) of Carbopol 940® and purified water (15g) were mixed uniformly by stirring, and diisopropanolamine (3.0g) was added to the mixture while continuing the stirring. To the gel base thus prepared, the alcoholic solution of diclofenac sodium previously prepared was added and the whole was adjusted to 100g by further adding purified water. After stirring well, a gel preparation having a viscosity of 17,000 centipoise and a pH of 7.20 was obtained.

Referential Example 1

Diclofenac sodium (1.0g) and peppermint oil (3.0g) were dissolved in 95% ethanol (40g) by stirring. Separately, 3% solution (20g) of Carbopol 940® in propylene glycol, 4% aqueous solution (25g) of Carbopol 940®, citric acid (0.3g) and purified water (8.0g) were mixed uniformly by stirring, and triethanolamine (0.05g) was added to the mixture while continuing the stirring. To the gel base thus prepared, the alcoholic solution of diclofenac sodium and peppermint oil prepared previously was added and the whole was adjusted to 100g by further addition of purified water. After stirring well, a gel preparation having a viscosity of 22,000 centipoise and a pH of 5.2 was obtained.

Referential Example 2

Diclofenac sodium (1.0g) was dissolved in 95% ethanol (40g) by stirring. Separately, 3% solution (20g) of Carbopol 940® in propylene glycol, 4% aqueous solution (25g) of Carbopol 940® and purified water (10g) were mixed uniformly by stirring, and triethanolamine (0.05g) was added to the mixture while continuing the stirring. To the gel base thus prepared, the alcoholic solution of diclofenac sodium prepared previously was added and the whole was adjusted to 100g by further addition of purified water. After stirring well, a gel preparation having a viscosity of 23,000 centipoise and a pH of 5.4 was obtained.

Referential Example 3

Diclofenac sodium (1.0g) was dissolved in 95% ethanol (20g) by stirring. Separately, 3% solution (40g) of Carbopol 940® in propylene glycol, 4% aqueous solution (10g) of Carbopol 940® and purified water (25g) were mixed uniformly by stirring, and triethanolamine (0.8g) was added to the mixture while continuing the stirring. To the gel base thus prepared, the alcoholic solution of diclofenac sodium previously prepared was added and the whole was adjusted to 100g by further adding purified water. After stirring well, a gel preparation having a viscosity of 21,000 centipoise and a pH of 5.8 was obtained.

Next, comparison between the gel preparations of the above Examples and the gel preparation of the Referential Examples was effected with regard to the stability of diclofenac sodium with the lapse of time. More precisely, the content of diclophenac sodium in each preparation was measured just after the preparation was obtained and after the lapse of a prescribed time, according to the method of analysis as described hereinafter. The changes with the lapse of time in the content of diclofenac sodium in the preparation of Example 1 are shown in Fig. 1, and those of the preparations of Referential Examples 1 to 3 are shown in Figs. 2 to 4, respectively (abscissa: days elapsed, ordinate: percentages of the active ingredient contained in the preparation, taking that just after the preparation is obtained as the 100%).

It is apparent from these Figures that the preparation of Example 1 is significantly high in the stability with the lapse of time, as compared with the preparations of the Referential Examples.

The measurement of the content of diclofenac sodium in the above gel preparation was effected by extracting diclofenac sodium with ethanol, isolating diclofenac sodium from the extract by high-speed liquid chromatography (column: Lichrosorb $RP_{18}$, developing agent: methanol/water/acetic acid - 600:400:5, room temperature), and determining the ultraviolet absorbance (254 nm).

## Claims

1. A gel preparation for external application characterized by comprising diclofenac sodium as the active ingredient and present in an amount of 0.3 to 3.0% by weight; a medium comprising water, ethanol, and a glycol selected from the group consisting of ethylene glycol, propylene glycol and 1,3-butylene glycol, the medium being present in an amount at least sufficient to dissolve the active ingredient, the ratio of water to the combination of the ethanol and glycol being from about 8:2 to 4:6; a carboxyvinyl polymer selected from the group consisting of the hydrophillic polymers obtained by polymerisation of acrylic acid, and present in an amount sufficient to gel the preparation; and an aliphatic amine, present in an amount sufficient to adjust the pH to about 6 to 8.

2. A gel preparation as claimed in claim 1, wherein the aliphatic amine is a primary, secondary or tertiary alkanolamine, or a primary, secondary or tertiary alkyl amine.

3. A gel preparation as claimed in claim 1, wherein the weak basic substance is triethanolamine or diisopropanolamine.

4. A gel preparation as claimed in claim 1, which further contains peppermint oil, l-menthol or salicylic acid ester added thereto as auxiliary agent.

5. A gel preparation as claimed in claim 4, wherein the salicylic acid ester is methyl salicylate, ethyl salicylate or glycol salicylate.

6. A gel preparation as claimed in claim 1, wherein the ratio by weight of water:ethanol:glycol in the medium is about 60:30:10.

7. The gel preparation of claim 1, wherein the aliphatic amine is present in an amount of from 0.1 to 5% by weight of the preparation.

## Patentansprüche

1. Gelpräparat für die externe Anwendung, dadurch gekennzeichnet, daß es Diclofenac-Natrium als aktiven Bestandteil und in einer Menge von 0,3 bis 3,0 Gew.-% vorhanden, ein Medium, welches Wasser, Ethanol und ein Glykol, ausgewählt aus der Gruppe Ethylenglykol, Propylenglykol und 1,3-Butylenglykol, enthält, wobei das Medium in einer Menge vorhanden ist, die mindestens ausreicht, den aktiven Bestandteil zu lösen, das Verhältnis von Wasser zu dem Gemisch aus Ethanol und Glykol von etwa 8:2 bis 4:6 beträgt, ein Carboxyvinyl-Polymeres, ausgewählt aus der Gruppe hydrophile Polymere, die durch Polymerisation von Acrylsäure erhalten werden und in einer Menge vorhanden sind, die ausreicht, das Präparat zu gelieren, und ein aliphatisches Amin, das in einer Menge vorhanden ist, die ausreicht, den pH auf etwa 6 bis 8 einzustellen, enthält.

2. Gelpräparat nach Anspruch 1, dadurch gekennzeichnet, daß das aliphatische Amin ein primäres, sekundäres oder tertiäres Alkanolamin oder ein primäres, sekundäres oder tertiäres Alkylamin ist.

3. Gelpräparat nach Anspruch 1, dadurch gekennzeichnet, daß die schwach basische Substanz Triethanolamin oder Diisopropanolamin ist.

4. Gelpräparat nach Anspruch 1, dadurch gekennzeichnet, daß es zusätzlich Pfefferminzöl, l-Menthol oder Salicylsäureester, welches als Hilfsmittel dazugegeben wird, enthält.

5. Gelpräparat nach Anspruch 4, dadurch gekennzeichnet, daß der Salicylsäureester Methylsalicylat, Ethylsalicylat oder Glykolsalicylat ist.

6. Gelpräparat nach Anspruch 1, dadurch gekennzeichnet, daß das Gewichtsverhältnis von Wasser:Ethanol:Glykol in dem Medium etwa 60:30:10 beträgt.

7. Gelpräparat nach Anspruch 1, dadurch gekennzeichnet, daß das aliphatische Amin in einer Menge von 0,1 bis 5 Gew.-%, bezogen auf das Präparat, vorhanden ist.

## Revendications

1. Préparation en gel pour application externe, caractérisée en ce qu'elle comprend du diclofénac sodique comme ingrédient actif, présent en une quantité de 0,3 à 3,0% en poids; un milieu comprenant de l'eau, de l'éthanol et un glycol choisi dans le groupe formé par l'éthylène-glycol, le propylène-glycol et le 1,3-butylène-glycol, le milieu étant présent en une quantité au moins suffisante pour dissoudre l'ingrédient actif, le rapport de l'eau à l'association de l'éthanol et du glycol étant d'environ 8:2 à 4:6; un polymère carboxyvinylique choisi dans le groupe formé par les polymères hydrophiles obtenus par polymérisation d'acide acrylique, présent en une quantité suffisante pour gélifier la préparation; et une amine aliphatique, présente en une quantité suffisante pour ajuster le pH à une valeur d'environ 6 à 8.

2. Préparation en gel telle que revendiquée dans la revendication 1, dans laquelle l'amine aliphatique est une alcanolamine primaire, secondaire ou tertiaire, ou une alkylamine primaire, secondaire ou tertiaire.

3. Préparation en gel telle que revendiquée dans la revendication 1, dans laquelle la substance faiblement basique est la triéthanolamine ou la diisopropanolamine.

4. Préparation en gel telle que revendiquée dans la revendication 1, qui contient en outre de l'essence de menthe poivrée, du l-menthol ou un ester d'acide salicylique ajouté comme agent auxiliaire.

5. Préparation en gel telle que revendiquée dans la revendication 4, dans laquelle l'ester d'acide salicylique est le salicylate de méthyle, le salicylate d'éthyle ou un salicylate de glycol.

6. Préparation en gel telle que revendiquée dans la revendication 1, dans laquelle le rapport en poids eau:éthanol:glycol dans le milieu est d'environ 60:30:10.

7. Préparation en gel selon la revendication 1, dans laquelle l'amine aliphatique est présente en une quantité de 0,1 à 5% en poids de la préparation.

FIG.1

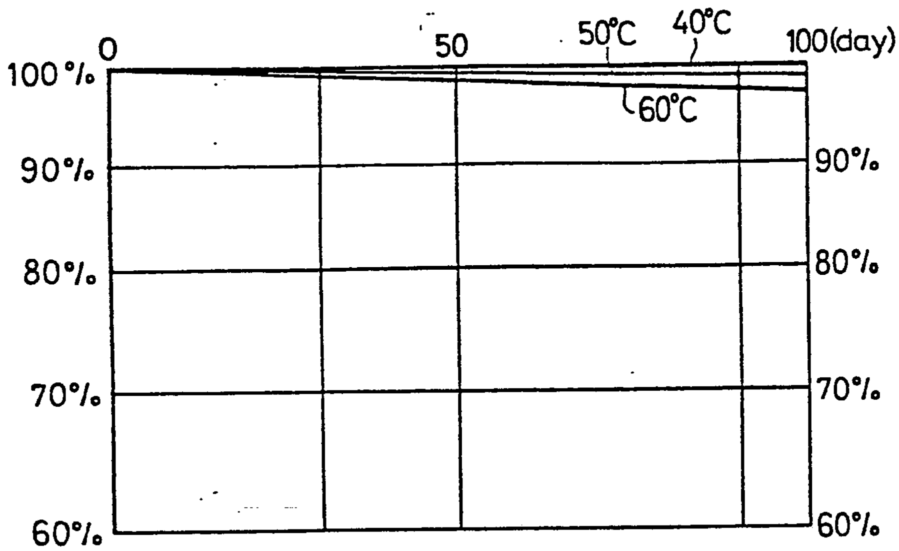
0
50
50°C
40°C
100(day)
100%
60°C
90%
90%
80%
80%
70%
70%
60%
60%

FIG.2

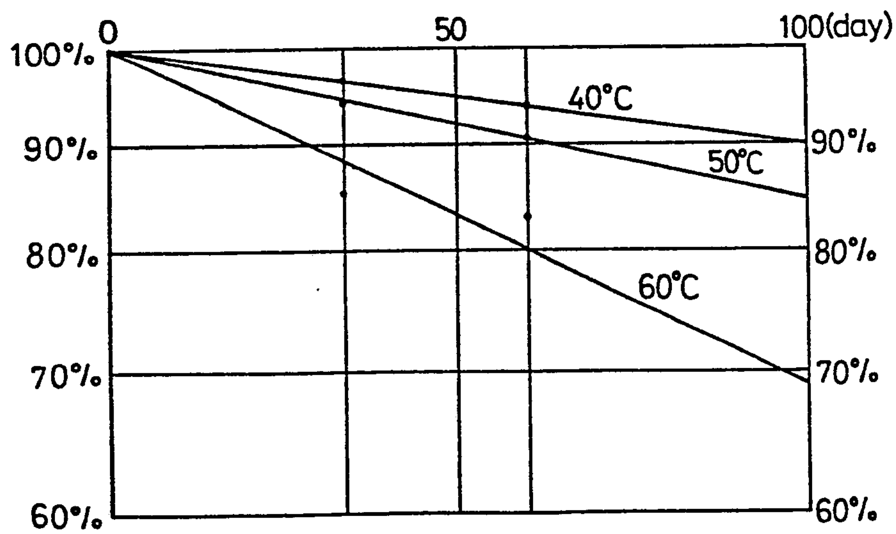
0
50
100(day)
100%
40°C
90%
90%
50°C
80%
80%
60°C
70%
70%
60%
60%

FIG. 3

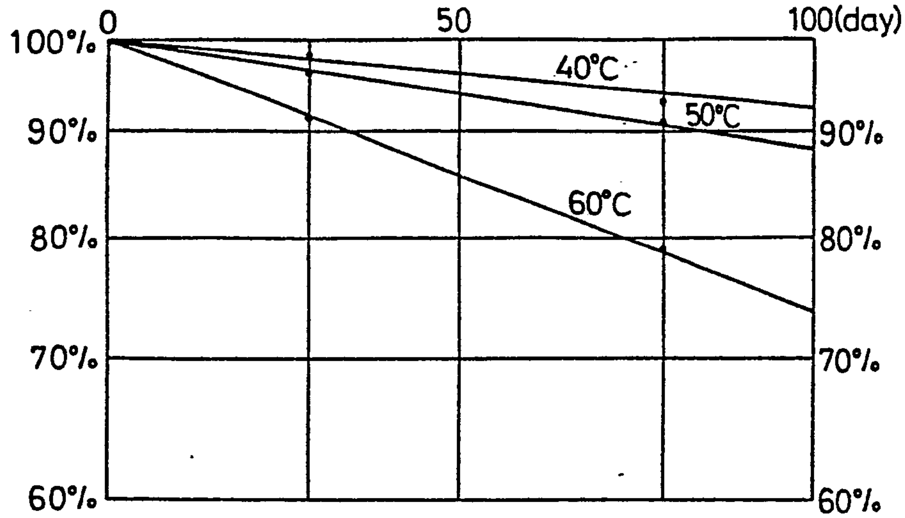
0
50
100(day)
100%
90%
80%
70%
60%
40°C
50°C
60°C
90%
80%
70%
60%

FIG. 4

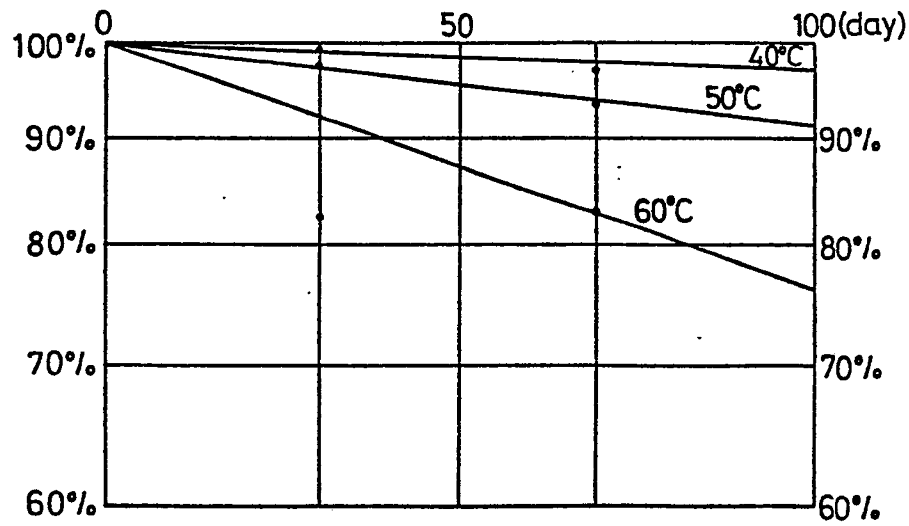
0
50
100(day)
100%
90%
80%
70%
60%
40°C
50°C
60°C
90%
80%
70%
60%